# EUROPEAN PATENT SPECIFICATION

(11) **EP 3 653 247 B1**
(45) Date of publication and mention of the grant of the patent: **31.03.2021**
(21) Application number: 18207022.7
(22) Date of filing: 19.11.2018
(51) Int. Cl.: A61M 15/00, G01F 1/34, G01F 15/06, G16H 20/13, G16H 40/63, G16H 40/67, A61M 16/00, A61M 16/08, A61B 5/00, A61B 5/087, G01F 15/14

(54) **DETERMINATION OF AIR FLOW RATE THROUGH AN INHALER**
BESTIMMUNG DER LUFTDURCHFLUSSMENGE DURCH EINEN INHALATOR
DÉTERMINATION D'UN DÉBIT D'AIR À TRAVERS UN INHALATEUR

(43) Date of publication of application: 20.05.2020
(73) Proprietor: Sensirion AG, 8712 Stäfa (CH)
(72) Inventor: ZIPKES, Christoph, 8712 Stäfa (CH); ALT, Andreas, 8712 Stäfa (CH)
(74) Representative: Detken, Andreas

(56) References cited:
- EP-A1- 0 667 168
- EP-A1- 2 582 468
- EP-A1- 3 338 842
- WO-A1-2017/178865
- WO-A1-2017/201463
- WO-A1-2017/205824
- US-A- 5 392 768
- US-A- 5 809 997
- US-A1- 2016 144 141
- US-A1- 2016 325 058
- US-A1- 2017 290 527
- US-B1- 6 752 145

## Description

### TECHNICAL FIELD

The present invention relates to an accessory for a medical inhaler, the accessory enabling the determination of an air flow rate through the inhaler. The invention further relates to an inhaler fitted with such an accessory, and to a method of determining an air flow rate through an inhaler using such an accessory.

### PRIOR ART

Medical inhalation devices (inhalers) are commonly used for delivering a drug in the form of an aerosol (i.e. a dispersion of fine solid particles or liquid droplets in air) to a patient's lungs. The most common types of inhalers are pressurized metered-dose inhalers (pMDIs) and dry-powder inhalers (DPIs).

A pMDI is disclosed, e.g., in US 2002/0144678 A1. The drug is stored in solution or suspension together with a propellant in a pressurized container. The container is closed by a metering valve. The container is held in a plastic housing defining an air inlet and an air outlet of the inhaler. At the air outlet, the housing forms a mouthpiece. The housing comprises a socket for receiving a valve stem of the metering valve. The socket defines a duct leading to a nozzle orifice located in the air flow path between the air inlet and the air outlet of the inhaler. In use, the patient inhales through the mouthpiece. This creates an air flow through the housing and past the nozzle orifice. After the patient has started inhaling, the patient depresses the container into the housing onto the valve stem, which is seated in the socket, thereby opening the metering valve and releasing a metered dose of the drug through the nozzle orifice. The released drug mixes with the air flow and is hence inhaled by the user through the mouthpiece. Usually, the complete pMDI is discarded after all the drug in the container has been consumed, i.e., the housing of the pMDI is normally not reused with a fresh drug container.

For optimum delivery of the drug, it is important that the patient, while inhaling, maintains an air flow in a certain desired range of flow rates, e.g., in a range of 30 to 60 l/min.

The creation of an air flow above a certain minimum flow rate or in a certain range of flow rates is also important for other types of inhalers. For instance, many dry-powder inhalers (DPIs) rely on the force of patient inhalation to break up the powder into particles that are small enough to reach the lung. It is therefore important that the patient maintains a sufficiently high air flow rate during inhalation.

There have been many suggestions in the prior art how patients can be trained to correctly create an air flow in a desired range of flow rates through an inhaler. F. Lavorini et al., "The ADMIT series - Issues in Inhalation Therapy. 6) Training tools for inhalation devices", Primary Care Respiratory Journal 2010, 335-341, doi: 10.4104/pcrj.2010.00065 discloses various training tools to this end. However, most of these devices cannot be integrated into actual inhalers and can only be used for training.

GB 2490770 A discloses an adapter for fitting to the mouthpiece of a pMDI. The adapter is a tube with an air flow rate indicator, to be attached to the outlet end of the mouthpiece so as to be interposed between the pMDI and the patient's mouth. The document further discloses an inhaler with an integrated electronic flow rate sensor. The flow rate sensor is located inside the inhaler body just upstream of the outlet of the device. The adapter of this document is disadvantageous in that it prolongs the flow path between the point where the drug is injected into the air flow and the mouth of the user. The inhaler with integrated electronic flow rate sensor is disadvantageous in that the flow rate sensor cannot be reused when the inhaler is discarded, and in that the flow rate sensor necessarily modifies the air flow through the device.

US 2017/0290527 A1 discloses a compliance monitoring module for an inhaler. The module comprises a pressure sensor having a pressure port that is pneumatically coupled to a flow channel of the inhaler.

US 5,392,768 discloses an inhaler that comprises a flow transducer system. The flow transducer system includes a pressure transducer.

US 2016/0144141 A1 discloses a detachable cap for measuring usage of an inhaler. The cap includes a pressure sensor adapted to detect air pressure within in the cap.

WO 2017/205824 A1 discloses an adapter that is attached to a medicament device. A sensor device comprising a barometer is attached to the adapter. The barometer is operably coupled to a pressure tap tube of the adapter. The barometer allows the sensor device to measure inhalation parameters.

WO 2011/157561 A1 discloses an inhaler that comprises a monitoring device. The monitoring device is arranged in a detachable housing part or fitted onto a mouthpiece of the inhaler. A supply air current is detected by means of a pressure sensor.

EP 3 338 842 A1 discloses an inhaler that comprises a registration means for determining a fluidic parameter of an aerosol when the aerosol is flowing through the mouthpiece.

### SUMMARY OF THE INVENTION

It is an object of the present invention to provide a device for determining an air flow rate through an inhaler, which can be reused and/or recycled separately from the inhaler, and which neither prolongs nor obstructs the aerosol flow through the inhaler downstream from the position where the drug is mixed with the air flow.

This object is achieved by an accessory according to claim 1, an inhalation system according to claim 9 and a method according to claim 12. The dependent claims 2-8, 10, 11 and 13-15 define preferred embodiments of the invention.

According to claim 1, an accessory for an inhaler is provided, the inhaler having an air inlet for the entry of air into the inhaler and an air outlet for communication with the mouth of a patient, such that inhalation by a patient through the air outlet causes an inhalation air flow through the inhaler from the air inlet to the air outlet. The inhaler further defines a mixing zone between the air inlet and the air outlet for mixing the inhalation air flow with a drug. The accessory comprises a fastening structure for fastening the accessory to the inhaler and an electronic sensor for determining a flow rate of the air flow. The accessory comprises a pressure port, and the fastening structure is configured to fasten the accessory to the inhaler in such a manner that the pressure port is arranged upstream from the air inlet of the inhaler and the mixing zone with respect to the inhalation air flow. The electronic sensor is in communication with the pressure port and is sensitive to a pressure change at the pressure port caused by the inhalation air flow. The pressure port is formed by a plurality of openings in the accessory housing, the openings being distributed circumferentially along a circumference of the air inlet of the inhaler when the accessory is fastened to the inhaler.

The present invention thus provides an attachment for an inhaler that enables a determination of the flow rate of the air flow through the inhaler. This allows direct conclusions about the manner in which the patient inhales the drug. Accordingly, feedback to the patient and/or information to medical personnel can be provided, based on the flow rate measurements by the accessory. The accessory is designed to be fastened to the outside of the inhaler, where it cannot disturb the aerosol flow. The pressure port of the attachment is arranged upstream from the point of injection of the drug into the air flow, where it cannot disturb the flow of the aerosol that is formed by mixing the released drug and the air flow. Since the flow of the aerosol is neither disturbed nor prolonged by the accessory, the accessory can be added to an existing inhaler without requiring new testing procedures or separate approval of the combination by health authorities. The accessory can be configured to be easily removable from the inhaler, such that it can be reused on a fresh inhaler and can be recycled separately from the inhaler once it has reached the end of its lifecycle.

The pressure port of the accessory is arranged such that an air flow that enters the inhaler through its air inlet causes a pressure change at the pressure port. The term "pressure port" is to be understood broadly as any structure that enables a direct or indirect determination of pressure at the location where the pressure port is situated, employing the electronic sensor. In the claimed invention, the pressure port is formed by a plurality of openings that enable direct gas exchange with the electronic sensor. Communication between the pressure port and the electronic sensor thus takes place pneumatically.

In particular, the accessory can be configured such that the air flow into the air inlet causes a pressure decrease (negative pressure) at the pressure port due to the flow velocity of the air flow. This effect is commonly called the Bernoulli effect or the Venturi effect.

In use of the accessory, the pressure port is located upstream from the mixing zone where the air flow and the drug are mixed to form an aerosol. Mixing of the air flow and the drug can be achieved, in particular, by releasing the drug into the air flow through an orifice, in particular, through a nozzle-type orifice, as it is the case in typical pMDIs. In this case, the mixing zone is a zone immediately downstream from the orifice. Accordingly, in this case the pressure port is arranged upstream from the orifice.

The inhaler can have an inhaler housing defining a receiving portion configured to receive a drug reservoir and an outlet portion forming a mouthpiece or being connected to a mouthpiece. The outlet portion and the receiving portion can extend at an angle to one another. Many pMDIs have such a configuration. The fastening structure can then be configured to fasten the accessory to the exterior of the receiving portion rather than to the exterior of the mouthpiece portion.

The fastening structure is configured to fasten the accessory to the exterior of the inhaler in such a manner that the pressure port is arranged upstream from the air inlet of the inhaler with respect to the air flow. In this manner it is possible to retrofit an existing inhaler with an accessory for flow rate determination without modification of the existing inhaler.

The accessory comprises an accessory housing, the electronic sensor being received in the accessory housing, and the pressure port is formed by a plurality of openings in the accessory housing, the openings being distributed circumferentially along a circumference of the air inlet of the inhaler across the direction of the air flow when the accessory is attached to the inhaler. In this manner, the total cross-sectional area of the pressure port can be increased without increasing the individual cross-sectional area of the individual openings, thereby reducing the risk of dust and liquids entering the housing through the openings and ensuring that the accessory remains functional even if one of the openings should get blocked.

In some embodiments, the pressure port is arranged in a wall portion of the accessory housing that is essentially flush with a housing wall of an inhaler housing when the accessory is fastened to the inhaler, the wall portion of the accessory housing and the housing wall of the inhaler housing delimiting a flow path of the air flow. In this manner, it can be ensured that the accessory will disturb the air flow that enters the inhaler only to the smallest possible degree.

More specifically, the accessory housing can define an axial stop structure configured to abut to a proximal end face of the housing wall when the accessory is fastened to the inhaler, and the wall portion in which the pressure port is arranged can be adjacent to the stop structure.

In some embodiments, the fastening structure comprises a ring configured to be arranged around an inhaler housing of the inhaler at the air inlet. In other embodiments, the fastening structure can comprise two prong-like arms that clasp the inhaler housing by the action of elastic forces. Many other ways of fastening the accessory to the inhaler housing are conceivable.

In alternative embodiments that are not part of the claimed invention, the accessory is configured in such a manner that the pressure port is arranged in or behind a through-opening in a wall of the inhaler housing when the accessory is fastened to the inhaler. For instance, the pressure port can be formed by a pipe that extends into the through-opening, or it can be formed by an opening in a wall portion of the accessory housing, said opening being arranged behind the through-opening in the wall of the inhaler housing.

In some embodiments, the pressure port can be arranged such that the air flow overflows the pressure port when the accessory is fastened to the inhaler. In other embodiments, the pressure port can be arranged such that the pressure port is shielded from direct air flow, for instance by being arranged in a recess of the accessory adjacent to the air flow.

In some embodiments, the accessory is configured to modify the air flow adjacent to the pressure port in such a manner that a reverse air flow due to exhalation by the patient through the inhaler causes a pressure change at the pressure port with opposite sign as compared to the pressure change caused by the air flow due to inhalation. In this manner, the accessory can distinguish between inhalation and exhalation. To this end, the accessory can comprise a flow-modifying structure that causes a positive dynamic pressure at the pressure port only for one of the two flow directions. For instance, the flow-modifying structure can comprise an obstruction upstream from the pressure port with respect to the air flow created by inhalation and downstream from the pressure port with respect to the reverse air flow created by exhalation, thereby causing a dynamic pressure at the pressure port only when the reverse air flow is present.

The electronic sensor can be any type of sensor that is capable of detecting the pressure change at the pressure port caused by the air flow. For instance, the electronic sensor can be a pressure sensor, in particular, an absolute pressure sensor (measuring pressure in comparison to absolute vacuum) or a differential pressure sensor (measuring pressure relative to some reference pressure, in particular, relative to the ambient pressure). A differential pressure sensor is sometimes also called a relative pressure sensor. Many different types of pressure sensors are known, based on different working principles, and the present invention is not limited to a particular type of pressure sensor. For instance, some types of pressure sensors comprise a deformable membrane, and the degree of deformation of the membrane by the air pressure is determined. This working principle is often employed in barometric pressure sensors, and the pressure sensor can accordingly be a barometric pressure sensor.

In some embodiments, the electronic sensor is a differential pressure sensor or a flow sensor. Some types of differential pressure sensors in fact work on the principle that a pressure difference causes a small flow through a flow path of the sensor, a sensing structure of the sensor being arranged adjacent to the flow path and being sensitive to the flow rate of that flow, and therefore a sharp distinction between flow sensors and this type of differential pressure sensor is sometimes not even possible. If the electronic sensor is a differential pressure sensor of the flow sensor type, it may have a first and a second sensor port. The first sensor port (or sensor inlet) will then be in fluid-tight communication with the pressure port of the accessory. The second sensor port (or sensor outlet) can be in communication with a reference port, the reference port being arranged such that the air flow does not cause a pressure change at the reference port, or being arranged such that the air flow causes a pressure change of different sign and/or magnitude at the reference port than at the pressure port.

The accessory can further comprise electronic circuitry connected to the electronic sensor, the electronic circuitry being operable to receive sensor signals from the electronic sensor and to derive an output signal based on the sensor signals. The accessory can comprise an output device for creating user feedback based on the output signal. The output device can comprise, e.g., a tone generator for creating acoustic feedback, a visual indicator such as one or more LEDs or an LCD screen for creating visual feedback, and/or a vibration generator for creating tactile feedback to the patient who is using the inhaler.

In some embodiments, the electronic circuitry is configured for wireless communication with a remote device. The remote device can be, for instance, a smartphone, a tablet computer, a notebook computer, or a remote server that is accessible via a wide-area network like the Internet. The electronic circuitry can comprise, for instance, a module enabling a wireless point-to-point link with the remote device, such as a Bluetooth™ module, or a module enabling network communication with the remote device via a wireless link, e.g. via a WiFi™ network or via a cellular network like a GSM network. The electronic circuitry can be configured to transmit the output signal to the remote device via the wireless link. In this manner, real-time feedback about the inhalation process can be provided to the patient, to medical personnel or to a remote analysis engine via the remote device. In addition or in the alternative, inhalation data can be stored in the remote device to be read out later.

According to claim 9, there is provided an inhalation system comprising an inhaler having an air inlet for the entry of air into the inhaler and an air outlet for communication with the mouth of a patient, the inhaler being configured such that inhalation by a patient through the air outlet causes an inhalation air flow through the inhaler from the air inlet to the air outlet, and further comprising an accessory as described above. The inhaler can in particular be a pMDI, a DPI, a nebulizer or a soft mist inhaler.

In particular, the inhaler can comprise a housing and a drug reservoir received in the housing, the drug reservoir being configured to release an aerosolized drug into the mixing zone when actuated by a patient. In particular, the housing can have an inlet end, the inlet end defining the air inlet of the inhaler, and an outlet end, the outlet end defining the air outlet of the inhaler. The outlet end can be shaped as a mouthpiece. In other embodiments, a separate mouthpiece is provided on the outlet end. The fastening structure of the accessory is advantageously configured to fasten the accessory to the housing of the inhaler. In particular, the fastening structure can be configured to fasten the accessory to the inhaler housing at its inlet end.

According to claim 12, there is provided a method consisting of the following step: fastening an accessory according to the invention to an inhaler having an air inlet for the entry of air into the inhaler and an air outlet for communication with the mouth of a patient, the inhaler defining a mixing zone between the air inlet and the air outlet for mixing an air flow through the inhaler from the air inlet to the air outlet with a drug; such that: the electronic sensor of the accessory is configured to measure a pressure parameter indicative of a pressure change at the pressure port caused by the air flow; and a flow rate parameter that is indicative of the flow rate through the inhaler can be determined based on the pressure parameter.

An output signal can be generated based on the flow rate parameter. The output signal can be a signal that essentially contains the flow rate parameter itself, or it can depend on the flow rate parameter in some other way. For instance, in a simple case, the output signal can be a binary signal, wherein a logical "1" indicates that the flow rate is within some predetermined limits, whereas a logical "0" indicates that the flow rate is outside these limits. In another embodiment, the output signal can a quantity that is calculated or otherwise derived from the flow rate, such as the total inhaled volume, the peak inspired flow, the airway resistance or other parameters. User feedback can be generated based on the output signal, e.g., acoustic, visual and/or tactile user feedback. The output signal can be transmitted to a remote device via a wireless link. The remote device can be operated to receive the transmitted output signal. The remote device can be operated, based on the received output signal, to generate user feedback for the patient.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows, in a highly schematic longitudinal section, an inhalation system comprising an inhaler and an accessory according to a first embodiment, together with a remote control device;
- Fig. 2: shows, in a highly schematic longitudinal section, a portion of an inhalation system comprising an inhaler together with an accessory according to a second embodiment;
- Fig. 3: shows, in a highly schematic perspective view, an accessory according to a third embodiment according to the invention;
- Fig. 4: shows, in a highly schematic longitudinal section, a portion of the accessory in Fig. 3; and
- Fig. 5: shows, in a highly schematic longitudinal section, an inhalation system comprising an inhaler and an accessory according to a fourth embodiment, which is not part of the claimed invention.

### DESCRIPTION OF PREFERRED EMBODIMENTS

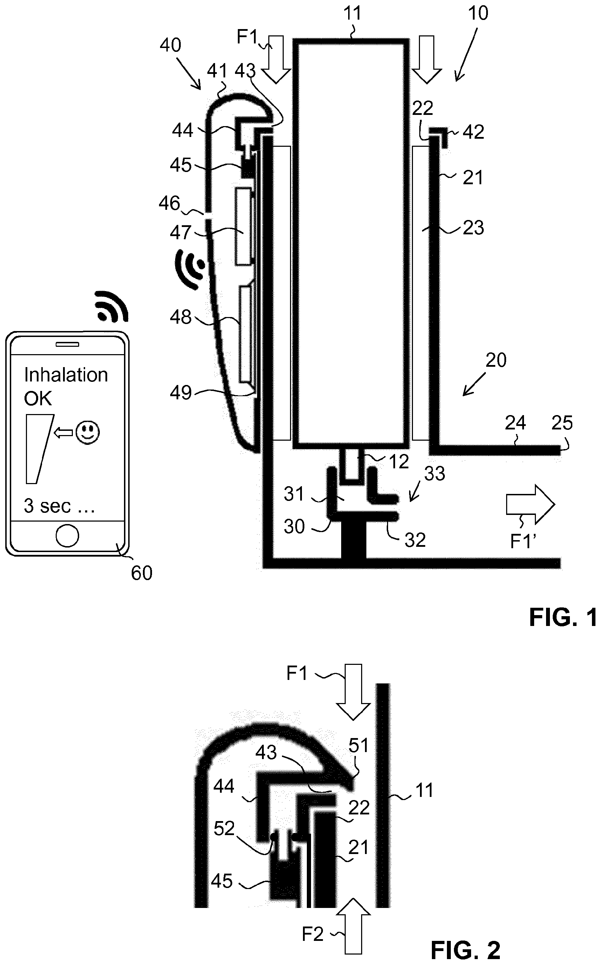

Fig. 1 illustrates, in a highly schematic manner, an inhalation system according to a first embodiment. The inhalation system comprises an inhaler 10 and an accessory 40. The accessory 40 is attached to the inhaler 10 by means of a fastening structure 42 so as to be removable from the inhaler 10.

In the present example, the inhaler 10 is a typical pMDI inhaler. It comprises a generally cylindrical drug reservoir or drug container 11 having at its lower end a metering valve for controlled release of single doses of a drug through a hollow valve stem 12. The metering valve is actuated by pressing the valve stem 12 into drug container 11.

The drug container 11 is received in an inhaler housing 20. The inhaler housing 20 is generally L-shaped. The upright leg of the L forms an upwardly open receiving portion for the drug container 10. The receiving portion has the shape of a hollow cylinder extending upwardly to an upper end 22. The receiving portion is defined by a circumferential housing wall 21. At the open upper end 22 of the receiving portion, the housing wall 21 forms an annular end face. The drug container 11 is held in the receiving portion by a plurality of spacer ribs 23 extending radially inwardly from the housing wall 21. In consequence, the outer circumferential wall of the drug container 11 is spaced from the housing wall 21 of the housing 20, and axially extending air channels are thus formed between the housing wall 21 and the drug container 11.

At the lower end of the receiving portion, the inhaler housing 20 includes a hollow socket 30. The valve stem 12 of the drug container 11 is seated in the socket 30. The socket 30 defines a duct 31 leading from the exit of the valve stem 12 to a nozzle orifice 32. The nozzle orifice 32 opens out into the interior of the inhaler housing 20 in a lateral direction that is transverse to the cylinder axis of the receiving portion, but not necessarily perpendicular to the cylinder axis.

The transverse leg of the L-shaped housing 20 extends in the same lateral direction as the nozzle orifice 32. At its far end, it forms a hollow mouthpiece 24 for insertion into the mouth of a patient. The mouthpiece 24 is laterally open at its end 25. It usually has a flattened cross-sectional shape adapted to the anatomy of a human patient's mouth. This cross-sectional shape is generally different from the cross-sectional shape of the receiving portion.

The open upper end 22 of the receiving portion of the inhaler housing 20 together with the circumferential wall of the drug container 11 forms an air inlet, allowing air to enter the inhaler. The open end 25 of the mouthpiece 24 forms an air outlet. A flow path exists through the inhaler housing 20 between the air inlet and the air outlet. The nozzle orifice 32 is disposed in this air flow path. Downstream from the nozzle orifice 32, the inhaler housing 20 defines a mixing zone 33 for mixing the air flow with the drug released from the drug container 11.

In use, a patient holds the inhaler 10 and applies his mouth to the mouthpiece 24. The patient then inhales through the mouthpiece 24, thereby creating an air flow F1 through the inhaler housing 20 from the air inlet to the air outlet. After the patient has started inhaling through the mouthpiece 14, the drug container 11 is pressed downwardly to release a dose of drug. Due to the pressure in the drug container 11, the drug is propelled through the duct 31 and the nozzle orifice 32 into the mixing zone 33, where it mixes with the air flow to form an aerosol flow F1', and is inhaled by the patient.

The accessory 40 comprises an accessory housing 41, which is held on the inhaler housing 20 by means of the fastening structure 42. In the present example, the fastening structure 42 forms a ring that is sled onto the open upper end of the receiving portion of the inhaler housing 20. At its upper end, the ring has an inwardly extending flange that rests on the upper end face of the housing wall 21 at the open upper end 22 of the receiving portion, thereby forming an axial stop structure together with the upper end 22 of the receiving portion. This ensures that the accessory housing 41 is fastened to the inhaler housing 20 in a defined axial position.

Inside the accessory housing 41, a carrier 49 in the form of a printed circuit board is disposed. The carrier 49 carries an electronic pressure sensor 45, electronic circuitry 47, and a battery 48. An opening in accessory housing 41 defines a pressure port 43. The electronic pressure sensor 45 communicates with the pressure port 43 pneumatically via a duct 44.

The pressure port 43 is arranged in an inwardly facing wall portion of the accessory housing 41 that is immediately adjacent to the open upper end 22 of the inhaler housing 20. The pressure port 43 is arranged such that the air flow F1, immediately before it enters the inhaler housing 20 at the air inlet, overflows the pressure port 43. The surface of the wall portion of the accessory housing 41 in which the pressure port 43 is arranged is flush with the inside surface of the housing wall 21 of the inhaler housing 20. These surfaces together delimit the air flow F1 at the air inlet. The accessory 40 of the first embodiment does not have any structure that projects into the air flow F1. Thereby it is ensured that the air flow F1 is disturbed as little as possible by the presence of the accessory 40.

In use, the air flow F1 caused by inhalation by the patient causes a negative pressure at the pressure port 43 due to the Bernoulli/Venturi effect caused by the acceleration of the air flow when it enters the inhaler. The magnitude of the negative pressure is directly related to the magnitude of the flow rate of the air flow F1. The negative pressure is registered by the electronic pressure sensor 45. The electronic circuitry 47 reads out the electronic pressure sensor 45 and determines a flow rate parameter from the sensor signals read out from the electronic pressure sensor 45. To this end, the electronic circuitry 47 may employ calibration data that relate measured pressure values to known flow rate values of air flow F1.

Based on the flow rate parameter, the electronic circuitry 47 generates an output signal. The output signal can be, e.g., a digital signal that directly represents the flow rate parameter. In other examples, the output signal can be a digital signal that indicates whether the flow rate parameter is within a predetermined range.

The electronic circuitry 47 can comprise structure for generating user feedback for the patient, based on the output signal. For instance, the electronic circuitry can include a tone generator to generate an audible signal that indicates to the patient whether the flow rate is within a desired range. As another example, the electronic circuitry can include a vibrator to generate a tactile signal in the form of a vibration pattern that indicates to the patient whether the flow rate is within a desired range. As yet another example, the electronic circuitry can include a display, e.g., an LCD display or one or more LEDs, to generate a visual signal that indicates to the patient whether the flow rate is within a desired range.

The electronic circuitry can comprise a wireless communication module for transmitting the output signal via a wireless link to a remote device 60. The remote device 60 can be, e.g., a smartphone, a tablet computer, or a notebook computer. In other embodiments, the remote device can be a dedicated device specifically configured for interaction with the accessory 40. In yet other embodiments, the remote device can be a remote server. The wireless communication module can be, e.g., a Bluetooth™ module for establishing a point-to-point link between the accessory 40 and the remote device 60, or it can be a WiFi™ module for connecting the accessory 40 to a wireless LAN that includes the remote device 60.

In the present example, the remote device 60 executes a computer program (an "app") that causes the remote device 60 to receive the output signal from the accessory 40 via the wireless link and to generate user feedback using an output device of the remote device. For instance, the app can cause the remote device to display user feedback and/or instructions for correct handling of the inhaler on a display screen of the remote device. In another example, the app can cause the remote device to output an audible signal, e.g., a voice message, via a loudspeaker of the remote device, instructing the user to handle the inhaler in a specific manner. In yet another example, the app can cause the remote device to provide tactile feedback, e.g., by vibrating, depending on the manner in which the patient handles the inhaler. This can all be done in real time.

In addition, the app can cause the remote device to store the received output signals for later readout, and/or to transmit the received output signals or quantities derived from the received output signals, such as statistical data, to a remote server for analysis. This enables the monitoring of the usage of the inhaler by medical personnel. In other embodiments, the output signals are directly transmitted from the attachment to a remote server for analysis.

The electronic pressure sensor 45 can, in particular, be a differential pressure sensor that is based on a flow measurement principle. A differential pressure sensor of this type has two sensor ports: a sensor inlet and a sensor outlet. A pressure difference between the sensor inlet and the sensor outlet causes a sensor gas flow through a sensor flow channel defined inside the differential pressure sensor. A flow-sensitive structure is arranged adjacent to the sensor flow channel for measuring a flow rate of the sensor gas flow through the sensor flow channel. Thus, a differential pressure sensor of this type essentially acts as a flow sensor that is configured to determine the pressure difference based on the determination of a flow rate through a flow channel between the sensor ports.

A suitable flow sensor that can be used to determine differential pressure is disclosed, e.g., in US 2016/0161314 A1. The inlet and outlet tubes of the flow sensor disclosed in this document can act as the sensor ports referred to in the present disclosure.

If a differential pressure sensor or flow sensor is used, one of the sensor ports of the sensor is pneumatically connected to the pressure port 43 in a fluid-tight manner. The other sensor port advantageously is pneumatically connected to the environment of the accessory 40 in a region that is not influenced by the air flow F1. To this end, the accessory housing 41 can comprise a reference port 46 in the form of an opening, the opening being arranged in a region of the accessory housing 41 that faces away from the inhaler. The opening causes the pressure inside the accessory housing 41 to be equal to the pressure of the environment of the accessory 40 in a region that is unaffected by the air flow F1. The sensor port that is not connected to the pressure port 43 can therefore be simply open towards the inside of accessory housing 41, without a fluid-tight connection being required between this sensor port and the reference port 46.

It should be noted that the accessory 40 of the first embodiment cannot distinguish between a flow F1 caused by inhalation through the inhaler and a reverse flow in the opposite direction, as it would be caused by exhalation through the inhaler.

Fig. 2 schematically illustrates a second embodiment that avoids this disadvantage. The second embodiment is largely identical to the first embodiment, and only a portion around the upper open end of the inhaler housing 20 is illustrated. Elements that have the same functionality as in the first embodiment are designated with the same reference signs as in Fig. 1.

A key difference of the second embodiment as compared to the first embodiment lies in the design of the accessory housing 41 in the immediate vicinity of the pressure port 43. Whereas in the first embodiment the pressure port 43 is arranged in a smooth wall portion of the accessory housing 41 that modifies the air flow F1 only minimally, in the second embodiment the accessory housing 41 comprises a flow-modifying structure 51 that deliberately projects into the flow path of the air flow F1 to modify the air flow. In the present example, the flow-modifying structure 51 acts as a local barrier immediately downstream from the pressure port 43 for a reverse air flow F2, thereby creating a positive dynamic pressure (velocity pressure) at the pressure port 43 for the reverse air flow F2. In this manner, the air flow F1 due to inhalation and the reverse air flow F2 due to exhalation can be readily distinguished by the sign of the pressure change at the pressure port 43. Also illustrated in Fig. 2 is the fluid-tight connection between the electronic sensor 45 and the duct 44 that leads to the pressure port 43, symbolized by a gasket 52.

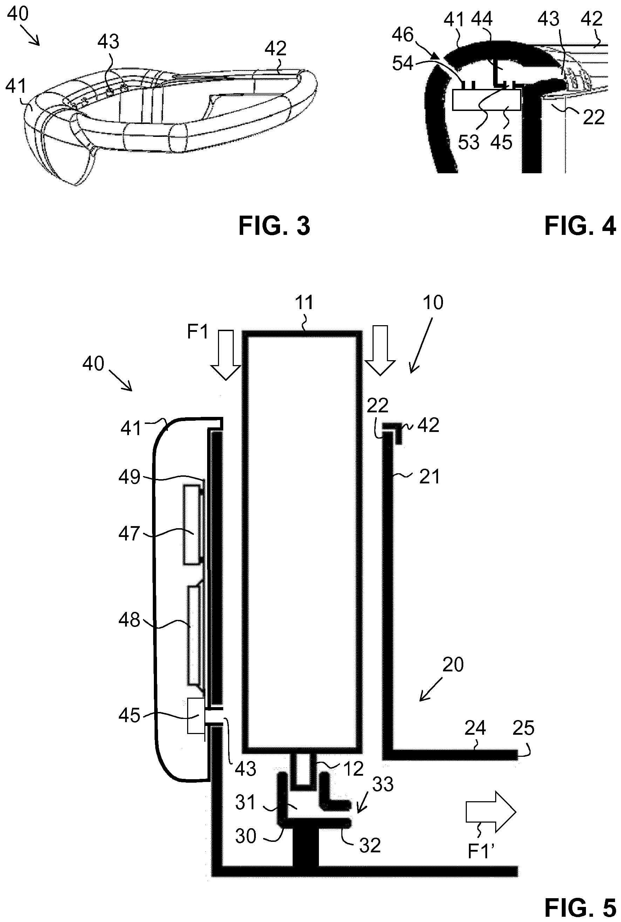

Figs. 3 and 4 illustrate a third embodiment of an accessory 40, according to the invention. Again, elements that have the same functionality as in the first embodiment are designated with the same reference signs as in Fig. 1.

As in the first and second embodiments, the fastening structure 42 is designed as a ring for attachment to the inlet end of the inhaler, more specifically, to the upper end 22 of the receiving portion of the inhaler housing. As in the first and second embodiments, the accessory housing 41 forms an axial stop with the upper end face of the inhaler housing to define the correct axial position of the accessory 40 on the inhaler housing. By the design of the fastening structure, the accessory 40 can only be attached to the inlet end of the inhaler, whereas it is impossible to accidentally fasten the accessory 40 to the outlet end, i.e., to the mouthpiece of the inhaler, due to its different shape.

The housing 41 of the accessory 40 according to the third embodiment has a plurality of openings, in particular, four openings, arranged along the circumference of the open end 22 of the receiving portion of the inhaler housing. As apparent from Fig. 4, all openings communicate pneumatically with a common duct 44, which in turn communicates pneumatically with the electronic sensor 45. In this manner, all openings together form the pressure port 43 of the third embodiment. Each opening has a sufficiently small individual cross-sectional area that dust or drops of liquid cannot easily enter the duct 44. At the same time, the openings together have a total cross-sectional area that gas exchange is possible between the inside and the outside of the duct 44 at a sufficiently high rate for ensuring proper operation of the electronic sensor 45.

As apparent from the schematic representation in Fig. 4, the electronic sensor 45 can be a differential pressure sensor or flow sensor, having two sensor ports 53, 54, the first sensor port being connected in a fluid-tight manner to the duct 44, while the second sensor port is in pneumatic communication with the environment via the reference port 46.

An accessory according to the third embodiment has been tested in conjunction with a commercially available pMDI. Measurements showed that an inhalation air flow of 60 l/min caused a pressure difference of about 20 Pa between the two sensor ports. A differential pressure of this magnitude can be readily detected by commercially available differential pressure sensors.

Fig. 5 illustrates a fourth embodiment of an accessory 40, which is not part of the claimed invention, together with a correspondingly configured inhaler 10. Again, elements that have the same functionality as in the first embodiment are designated with the same reference signs as in Fig. 1.

As in the first to third embodiments, the accessory 40 is attached to the outside of the inhaler housing 20 and can be easily removed from the inhaler. In the fourth embodiment, the circumferential wall 21 of the inhaler housing 20 is provided with a through-hole upstream from nozzle orifice 32, and the pressure port 43 is formed by the open end of a short pipe that extends into this through-hole to measure the flow rate of the air flow F1 without significantly disturbing the air flow. By arranging the pressure port 43 upstream of the nozzle orifice 32 with respect to the inhalation air flow F1, it is ensured that the electronic sensor 45 cannot be contaminated with the drug that is released through the nozzle orifice as long as the inhaler is used properly.

In a modification of the fourth embodiment (not illustrated in the drawings), the accessory 40 or the inhaler housing 20 is provided with a flow-modifying structure adjacent to the pressure port 43 inside the inhaler housing 20 in order to be able to distinguish between inhalation and exhalation, as discussed above in conjunction with the second embodiment.

While the accessory of the first to third embodiment can be fitted to any existing inhaler, the inhaler of the fourth embodiment may require adaptation to the intended use by providing the inhaler housing 20 with the through-hole for the pressure port 43.

While exemplary embodiments of the invention have been illustrated with reference to the drawings, the invention is by no means limited to these embodiments, and many modifications are possible without departing from the scope of the present claims. For instance, the electronic sensor can be of a different type than described above. In particular, the electronic sensor can be any other type of pressure sensor, for instance an absolute pressure sensor or a relative pressure sensor that is based on a different measurement principle than a flow measurement. In particular, the pressure sensor can be a barometric pressure sensor. The accessory can be fastened to the inhaler in a different manner than described. For instance, the accessory can be clamped to the inhaler body by two prong-like arms. The accessory of the present invention can also be used with other types of inhalers than the pMDI shown.

**LIST OF REFERENCE SIGNS**

| | | | |
|---|---|---|---|
| 10 | inhaler | 43 | pressure port |
| 11 | drug container | 44 | duct |
| 12 | valve stem | 45 | electronic sensor |
| 20 | inhaler housing | 46 | reference port |
| 21 | housing wall | 47 | circuitry |
| 22 | upper end | 48 | battery |
| 23 | spacer rib | 49 | carrier |
| 24 | mouthpiece | 51 | flow-modifying structure |
| 25 | air outlet | 52 | gasket |
| 30 | socket | 53, 54 | sensor port |
| 31 | duct | 60 | remote device |
| 32 | nozzle orifice | F1 | air flow |
| 33 | mixing zone | F1' | aerosol flow |
| 40 | accessory | F2 | reverse air flow |
| 41 | accessory housing | | |
| 42 | fastening structure | | |

## Claims

1. An accessory (40) for an inhaler (10), the inhaler (10) having an air inlet for the entry of air into the inhaler (10) and an air outlet for communication with the mouth of a patient, such that inhalation by a patient through the air outlet causes an inhalation air flow (F1) through the inhaler (10) from the air inlet to the air outlet, the inhaler (10) further defining a mixing zone (33) between the air inlet and the air outlet for mixing the inhalation air flow (F1) with a drug, the accessory (40) comprising
an accessory housing (41);
a pressure port (43);
an electronic sensor (45) for determining a flow rate of the inhalation air flow (F1), the electronic sensor (45) being received in the accessory housing (41), the electronic sensor (45) being in communication with the pressure port (43), the electronic sensor (45) being sensitive to a pressure change at the pressure port (43) caused by the inhalation air flow (F1); and
a fastening structure (42) for fastening the accessory (40) to the inhaler (10), the fastening structure (42) being configured to fasten the accessory (40) to an exterior of the inhaler (10) in such a manner that the pressure port (43) is arranged upstream from the air inlet of the inhaler (10) and the mixing zone (33) with respect to the inhalation air flow (F1),
**characterized in that** the pressure port (43) is formed by a plurality of openings in the accessory housing (41), the openings being distributed circumferentially along a circumference of the air inlet of the inhaler (10) when the accessory (40) is fastened to the inhaler (10).

2. The accessory (40) of claim 1, being configured to modify the inhalation air flow (F1) adjacent to the pressure port (43) in such a manner that a reverse air flow (F2) due to exhalation by the patient through the inhaler (10) causes a pressure change at the pressure port (43) with opposite sign as compared to the pressure change caused by the inhalation air flow (F1) due to inhalation, wherein the modification of the inhalation air flow (F1) is caused by a flow-modifying structure of the accessory causing a positive dynamic pressure at the pressure port (43) for only one of the flow directions of the inhalation air flow (F1) and the reverse air flow (F2).

3. The accessory (40) of claim 1 or 2, wherein the accessory housing (41) defines an axial stop structure configured to abut to a proximal end face of a housing wall (21) of a housing (20) of the inhaler (10) when the accessory (40) is fastened to the inhaler (10), and wherein a wall portion of the accessory housing (41) in which the pressure port (43) is arranged is adjacent to the stop structure.

4. The accessory (40) of any one of the preceding claims, wherein the fastening structure (42) comprises a ring configured to be arranged around an inhaler housing (20) of the inhaler (10).

5. The accessory (40) of any one of the preceding claims, wherein the electronic sensor is an absolute pressure sensor, a barometric pressure sensor, a differential pressure sensor or a flow sensor.

6. The accessory (40) of any one of the preceding claims,
wherein the electronic sensor (45) is a differential pressure sensor or a flow sensor, the electronic sensor having a first and a second sensor port (53, 54), the first sensor port (53) being in communication with the pressure port (43),
wherein the accessory (40) comprises a reference port (46), the second sensor port (54) being in communication with the reference port (46), and
wherein the reference port (46) is arranged such that the inhalation air flow (F1) does not cause a pressure change at the reference port (46) or causes a pressure change of different sign and/or magnitude at the reference port (46) than at the pressure port (43) when the accessory (40) is fastened to the inhaler (10).

7. The accessory (40) of any one of the preceding claims, further comprising electronic circuitry (47) connected to the electronic sensor (45), the electronic circuitry (47) being operable to receive sensor signals from the electronic sensor (45) and to derive an output signal based on the sensor signals.

8. The accessory of claim 7, wherein the electronic circuitry (47) is configured to transmit the output signal via a wireless link to a remote device (60).

9. An inhalation system comprising:
an inhaler (10) having an air inlet for the entry of air into the inhaler (10) and an air outlet for communication with the mouth of a patient, the inhaler (10) being configured such that inhalation by a patient through the air outlet causes an inhalation air flow (F1) through the inhaler (10) from the air inlet to the air outlet, the inhaler (10) further defining a mixing zone between the air inlet and the air outlet for mixing the air flow with a drug,
wherein the inhalation system further comprises an accessory (40) according to any one of the preceding claims.

10. The inhalation system of claim 9, wherein the inhaler (10) further comprises:
an inhaler housing (20); and
a drug reservoir (11) received in the inhaler housing (20), the drug reservoir (11) being configured to release an aerosolized drug into an air flow path between the air inlet and the air outlet,
wherein the fastening structure (42) of the accessory (40) is configured to fasten the accessory (40) to the inhaler housing (20).

11. The inhalation system of claim 9 or 10, wherein the pressure port (43) of the accessory is arranged in a wall portion of the accessory housing (41) of the accessory that is essentially flush with a housing wall of the inhaler housing (20) when the accessory (40) is fastened to the inhaler (10), the wall portion of the accessory housing (41) and the housing wall of the inhaler housing (20) delimiting a flow path of the inhalation air flow.

12. A method consisting of the following step:
fastening an accessory according to any one of claims 1 to 8 to an inhaler (10) having an air inlet for the entry of air into the inhaler (10) and an air outlet for communication with the mouth of a patient, the inhaler defining a mixing zone (33) between the air inlet and the air outlet for mixing air flow through the inhaler from the air inlet to the air outlet with a drug;
such that:
the electronic sensor (45) of the accessory (40) is configured to measure a pressure parameter indicative of a pressure change at the pressure port (43) caused by the air flow (F1); and
a flow rate parameter that is indicative of the flow rate through the inhaler (10) can be determined based on the pressure parameter.

13. The method of claim 12, wherein the accessory (40) comprises electronic circuitry (47) connected to the electronic sensor (45), and the electronic circuitry (47) is configured to determine the flow rate parameter.

14. The method of claim 13, wherein the electronic circuitry (47) is further configured to: based on the determined flow rate parameter, generate an output signal and transmit the generated output signal to a remote device (60) via a wireless link; such that the remote device (60) can be operated to receive the transmitted output signal.

## Patentansprüche

1. Zubehör (40) für einen Inhalator (10), wobei der Inhalator (10) einen Lufteinlass für den Eintritt von Luft in den Inhalator (10) und einen Luftauslass zur Kommunikation mit dem Mund eines Patienten aufweist, so dass die Inhalation durch einen Patienten durch den Luftauslass einen Inhalationsluftstrom (F1) durch den Inhalator (10) vom Lufteinlass zum Luftauslass bewirkt, wobei der Inhalator (10) weiter eine Mischzone (33) zwischen dem Lufteinlass und dem Luftauslass zum Mischen des Inhalationsluftstroms (F1) mit einem Medikament definiert, wobei das Zubehör umfasst:
ein Zubehörgehäuse (41);
einen Druckanschluss (43);
einen elektronischen Sensor (45) zur Bestimmung einer Durchflussrate des Inhalationsluftstroms (F1), wobei der elektronische Sensor (45) im Zubehörgehäuse (41) aufgenommen ist, wobei der elektronische Sensor (45) in Kommunikation mit dem Druckanschluss (43) ist, wobei der elektronische Sensor (45) auf eine Druckänderung am Druckanschluss (43) reagiert, die durch den Inhalationsluftstrom (F1) erzeugt wird; und
eine Befestigungsstruktur (42) zur Befestigung des Zubehörs (40) am Inhalator (10), wobei die Befestigungsstruktur (42) dazu ausgebildet ist, das Zubehör (40) an einer Aussenseite des Inhalators (10) in einer Weise zu befestigen, so dass der Druckanschluss (43) stromaufwärts vom Lufteinlass des Inhalators (10) und der Mischzone (33) bezüglich dem Inhalationsluftstrom (F1) angeordnet ist,
**dadurch gekennzeichnet, dass** der Druckanschluss (43) durch eine Vielzahl von Öffnungen im Zubehörgehäuse (41) gebildet ist, wobei die Öffnungen umfangsmässig entlang eines Umfangs des Lufteinlasses des Inhalators (10) verteilt sind wenn das Zubehör (40) am Inhalator (10) befestigt ist.

2. Zubehör (40) gemäss Anspruch 1, welches ausgebildet ist, um den Inhalationsluftstrom (F1) neben dem Druckanschluss (43) derart zu modifizieren, dass ein umgekehrter Luftstrom (F2) aufgrund der Ausatmung des Patienten durch den Inhalator (10) eine Druckänderung am Druckanschluss (43) mit entgegengesetztem Vorzeichen im Vergleich zur Druckänderung aufgrund des Inhalationsluftstroms (F1) durch die Inhalation erzeugt wird, wobei die Modifikation des Inhalationsluftstroms (F1) durch eine strömungsverändernde Struktur des Zubehörs (40) verursacht wird, welche einen positiven dynamischen Druck am Druckanschluss (43) für nur eine der Strömungsrichtungen des Inhalationsluftstroms (F1) und des umgekehrten Luftstroms (F2) bewirkt.

3. Zubehör (40) gemäss Anspruch 1 oder 2, wobei das Zubehörgehäuse (41) eine axiale Anschlagstruktur definiert, die dazu ausgebildet ist, gegen eine proximale Endfläche einer Gehäusewand (21) eines Gehäuses (20) des Inhalators (10) anzuschlagen, wenn das Zubehör (40) am Inhalator (10) befestigt ist, und wobei ein Wandabschnitt des Zubehörgehäuses (41) in welchem der Druckanschluss (43) angeordnet ist, and die Anschlagstruktur angrenzt.

4. Zubehör (40) gemäss einem der vorhergehenden Ansprüche, wobei die Befestigungsstruktur (42) einen Ring umfasst, der derart ausgebildet ist, dass er um ein Inhalatorgehäuse (20) des Inhalators herum angeordnet werden kann.

5. Zubehör (40) gemäss einem der vorhergehenden Ansprüche, wobei der elektronische Sensor ein Absolutdrucksensor, ein barometrischer Drucksensor, ein Differenzdrucksensor oder ein Durchflusssensor ist.

6. Zubehör (40) gemäss einem der vorhergehenden Ansprüche,
wobei der elektronische Sensor (45) ein Differenzdrucksensor oder ein Durchflusssensor ist, wobei der elektronische Sensor einen ersten und einen zweiten Sensoranschluss (53, 54) umfasst, wobei der erste Sensoranschluss (53) in Kommunikation mit dem Druckanschluss (43) ist,
wobei das Zubehör (40) einen Referenzanschluss (46) umfasst, wobei der zweite Sensoranschluss (54) in Kommunikation mit dem Referenzanschluss (46) ist, und
wobei der Referenzanschluss (46) derart angeordnet ist, dass der Inhalationsluftstrom (F1) keine Druckänderung am Referenzanschluss (46) bewirkt oder eine Druckänderung mit einem anderen Vorzeichen und/oder Grösse am Referenzanschluss (46) als am Druckanschluss (43) verursacht wenn das Zubehör (40) am Inhalator (10) befestigt ist.

7. Zubehör (40) gemäss einem der vorhergehenden Ansprüche, weiter umfassend eine elektronische Schaltung (47), die mit dem elektronischen Sensor (45) verbunden ist, wobei die elektronische Schaltung betreibbar ist, um Sensorsignale vom elektronischen Sensor (45) zu empfangen und um ein Ausgangssignal basierend auf den Sensorsignalen abzuleiten.

8. Zubehör (40) gemäss Anspruch 7, wobei die elektronische Schaltung (47) dazu ausgebildet ist, das Ausgangssignal über eine drahtlose Verbindung an ein entferntes Gerät (60) zu übertragen.

9. Inhalationssystem umfassend:
einen Inhalator (10) mit einem Lufteinlass für den Eintritt von Luft in den Inhalator (10) und einen Luftauslass zur Kommunikation mit dem Mund eines Patienten, wobei der Inhalator (10) derart ausgebildet ist, dass die Inhalation durch einen Patienten durch den Luftauslass einen Inhalationsluftstrom (F1) durch den Inhalator (10) vom Lufteinlass zum Luftauslass bewirkt, wobei der Inhalator (10) weiter eine Mischzone (33) zwischen dem Lufteinlass und dem Luftauslass zum Mischen des Luftstroms mit einem Medikament definiert,
wobei das Inhalationssystem weiter ein Zubehör (40) gemäss einem der vorhergehenden Ansprüche umfasst.

10. Inhalationssystem gemäss Anspruch 9, wobei der Inhalator (10) weiter umfasst:
ein Inhalatorgehäuse (20); und
ein im Inhalatorgehäuse (20) aufgenommenes Medikamentenreservoir (11), wobei das Medikamentenreservoir (11) dazu ausgebildet ist, ein aerosoliertes Medikament in einen Luftströmungsweg zwischen dem Lufteinlass und dem Luftauslass abzugeben,
wobei die Befestigungsstruktur (42) des Zubehörs (40) ausgebildet ist, das Zubehör (40) am Inhalatorgehäuse (20) zu befestigen.

11. Inhalationssystem gemäss Anspruch 9 oder 10, wobei der Druckanschluss (43) des Zubehörs in einem Wandabschnitt des Zubehörgehäuses (41) des Zubehörs angeordnet ist, der im Wesentlichen bündig mit einer Gehäusewand des Inhalatorgehäuses (20) ist, wenn das Zubehör (40) am Inhalator (10) befestigt ist, wobei der Wandabschnitt des Zubehörgehäuses (41) und die Gehäusewand des Inhalatorgehäuses (20) einen Strömungsweg des Inhalationsluftstroms begrenzen.

12. Verfahren bestehend aus den folgenden Schritten:
Befestigen eines Zubehörs gemäss einem der Ansprüche 1 bis 8 an einem Inhalator (10) umfassend einen Lufteinlass zum Eintritt von Luft in den Inhalator (10) und einen Luftauslass zur Kommunikation mit dem Mund eines Patienten, wobei der Inhalator (10) eine Mischzone (33) zwischen dem Lufteinlass und dem Luftauslass zum Mischen eines Luftstroms durch den Inhalator vom Lufteinlass zum Luftauslass mit einem Medikament definiert, so dass:
der elektronische Sensor (45) des Zubehörs (40) dazu ausgebildet ist, einen Druckparameter zu messen, der indikativ für eine Druckänderung am Druckanschluss (43) aufgrund des Luftstroms (F1) ist; und
ein Durchflussparameter indikativ für die Durchflussrate durch den Inhalator (10) basierend auf dem Druckparameter bestimmbar ist.

13. Verfahren gemäss Anspruch 12, wobei das Zubehör (40) eine elektronische Schaltung (47) umfasst, die mit dem elektronischen Sensor (45) verbunden ist, und wobei die elektronische Schaltung (47) dazu ausgebildet ist, den Durchflussparameter zu bestimmen.

14. Verfahren gemäss Anspruch 13, wobei die elektronische Schaltung (47) weiter dazu ausgebildet ist:
ein Ausgangssignal basierend auf dem bestimmten Durchflussparameter zu generieren und das generierte Ausgangssignal an ein entferntes Gerät (60) über eine drahtlose Verbindung zu übermitteln, so dass das entfernte Gerät (60) zum Empfang des übertragenen Ausgangssignals betreibbar ist.

## Revendications

1. Un accessoire (40) pour un inhalateur (10), l'inhalateur (10) ayant une entrée d'air pour l'entrée d'air dans l'inhalateur (10) et une sortie d'air pour communiquer avec la bouche d'un patient, de sorte que l'inhalation par un le patient à travers la sortie d'air provoque un flux d'air d'inhalation (F1) à travers l'inhalateur (10) de l'entrée d'air à la sortie d'air, l'inhalateur (10) définissant en outre une zone de mélange (33) entre l'entrée d'air et la sortie d'air pour mélanger le flux d'air (F1) avec un médicament, l'accessoire (40) comprenant:
un boitier d'accessoire (41);
un orifice de pression (43);
un capteur électronique (45) pour déterminer un débit du flux d'air d'inhalation (F1), le capteur électronique (45) étant logé dans le boitier d'accessoire (41), le capteur électronique (45) étant en communication avec l'orifice de pression (43), le capteur électronique (45) étant sensible à un changement de pression au niveau de l'orifice de pression (43) provoqué par le flux d'air d'inhalation (F1); et
une structure de fixation (42) pour fixer l'accessoire (40) à l'extérieur de l'inhalateur (10), la structure de fixation (42) étant configurée pour fixer l'accessoire (40) à un extérieur de l'inhalateur (10) de telle manière que l'orifice de pression (43) est disposé en amont de l'entrée d'air de l'inhalateur (10) et de la zone de mélange (33) par rapport au flux d'air d'inhalation (F1),
**caractérisé en ce que** l'orifice de pression (43) est formé par une pluralité d'ouvertures dans le boîtier accessoire (41), les ouvertures étant distribuées de manière circonférentielle le long d'une circonférence de l'entrée d'air de l'inhalateur (10) lorsque l'accessoire (40) est fixé à l'inhalateur (10).

2. L'accessoire (40) selon la revendication 1, étant configuré pour modifier le flux d'air d'inhalation (F1) adjacent à l'orifice de pression (43) de telle manière qu'un flux d'air inversé (F2) dû à l'expiration du patient à travers l'inhalateur (10) provoque un changement de pression au niveau de l'orifice de pression (43) avec un signe opposé par rapport au changement de pression provoqué par le flux d'air d'inhalation (F1) dû à l'inhalation, dans lequel la modification du flux d'air d'inhalation (F1) est engendrée par une structure modifiant le flux de l'accessoire causant une pression dynamique positive au niveau de l'orifice de pression (43) pour uniquement une des directions de flux du flux d'air d'inhalation (F1) et du flux d'air inversé (F2).

3. L'accessoire selon la revendication 1 ou 2, dans lequel le boitier d'accessoire (41) définit une structure d'arrêt axiale configurée pour venir en butée avec une face d'extrémité proximale d'une paroi de boîtier (21) d'un boîtier (20) de l'inhalateur (10), lorsque l'accessoire (40) est fixé à l'inhalateur (10), et dans lequel une partie de la paroi du boitier d'accessoire (41) dans lequel l'orifice de pression (43) est disposé est adjacent à la structure d'arrêt.

4. L'accessoire (40) selon l'une quelconque des revendications précédentes, dans lequel la structure de fixation (42) comprend un anneau configuré pour être agencé autour d'un boîtier d'inhalateur (20) de l'inhalateur (10).

5. L'accessoire (40) selon l'une quelconque des revendications précédentes, dans lequel le capteur électronique est un capteur de pression absolue, un capteur de pression barométrique, un capteur de pression différentielle ou un capteur de flux.

6. L'accessoire (40) selon l'une quelconque des revendications précédentes,
dans lequel le capteur électronique (45) est un capteur de pression différentielle ou un capteur de flux, le capteur électronique ayant un premier et un second orifice de capteur (53, 54), le premier orifice de capteur (53) étant en communication avec l'orifice de pression (43),
dans lequel l'accessoire (40) comprend un orifice de référence (46), le second orifice de capteur (54) étant en communication avec l'orifice de référence (46), et
dans lequel l'orifice de référence (46) est agencé de telle sorte que le flux d'air d'inhalation (F1) ne provoque pas de changement de pression au niveau de l'orifice de référence (46) ou provoque un changement de pression de signe et / ou de grandeur différents au niveau de l'orifice de référence (46) qu'au niveau de l'orifice de pression (43) lorsque l'accessoire (40) est fixé à l'inhalateur (10).

7. L'accessoire (40) selon l'une quelconque des revendications précédentes, comprenant en outre un circuits électronique (47) connecté au capteur électronique (45), le circuit électronique (47) étant opérables pour recevoir des signaux de capteur du capteur électronique (45) et pour dériver un signal de sortie basé sur les signaux du capteur.

8. L'accessoire selon la revendication 7, dans lequel le circuit électronique (47) est configuré pour transmettre le signal de sortie via une liaison sans fil à un dispositif distant (60).

9. Un système d'inhalation comprenant:
un inhalateur (10) ayant une entrée d'air pour l'entrée d'air dans l'inhalateur (10) et une sortie d'air pour communiquer avec la bouche d'un patient, l'inhalateur (10) étant configuré de telle sorte que l'inhalation par un patient à travers la sortie d'air provoque un flux d'air d'inhalation (F1) à travers l'inhalateur (10) de l'entrée d'air à la sortie d'air, l'inhalateur (10) définissant en outre une zone de mélange entre l'entrée d'air et la sortie d'air pour mélanger le flux d'air avec un médicament,
dans lequel le système d'inhalation comprend en outre un accessoire (40) selon l'une quelconque des revendications précédentes.

10. Le système d'inhalation selon la revendication 9, dans lequel l'inhalateur (10) comprend en outre:
un boîtier d'inhalateur (20); et
un réservoir de médicament (11) logé dans le boîtier d'inhalateur (20), le réservoir de médicament (11) étant configuré pour libérer un médicament en aérosol dans un trajet de flux d'air entre l'entrée d'air et la sortie d'air,
dans lequel la structure de fixation (42) de l'accessoire (40) est configurée pour fixer l'accessoire (40) au boîtier d'inhalateur (20).

11. Le système d'inhalation de la revendication 9 ou 10, dans lequel l'orifice de pression (43) de l'accessoire est disposé dans une partie de la paroi du boitier d'accessoire (41) de l'accessoire qui est sensiblement cohérente avec la paroi de boitier de l'inhalateur du boitier d'inhalateur (20) lorsque l'accessoire (40) est fixé à l'inhalateur (10), la partie de la paroi du boitier d'accessoire (41) et la paroi de boitier du boitier d'inhalateur (20) délimitant un trajet de flux du flux d'air d'inhalation.

12. Un procédé consistant de l'étape suivante:
fixer un accessoire selon l'une quelconque des revendications 1 à 8 à un inhalateur (10) ayant une entrée d'air pour l'entrée d'air dans l'inhalateur (10) et une sortie d'air pour communiquer avec la bouche d'un patient, l'inhalateur définissant une zone de mélange (33) entre l'entrée d'air et la sortie d'air pour mélanger un flux d'air à travers l'inhalateur de l'entrée d'air vers la sortie d'air avec un médicament; de sorte que:
le capteur électronique (45) de l'accessoire (40) est configuré pour mesurer un paramètre de pression indicatif d'un changement de pression au niveau de l'orifice de pression (43) provoqué par l'écoulement d'air (F1); et
un paramètre de débit qui est indicatif du débit à travers l'inhalateur (10) peut être déterminé sur la base du paramètre de pression.

13. Le procédé selon la revendication 12 dans lequel l'accessoire (40) comprend un circuit électronique (47) connecté au capteur électronique (45), et le circuit électronique (47) est configuré pour déterminer un paramètre de débit.

14. Le procédé selon la revendication 13 dans lequel le circuit électronique (47) est configuré en outre pour :
sur la base du paramètre de débit déterminé, générer un signal de sortie et
transmettre le signal de sortie généré à un dispositif distant (60) via une liaison sans fil;
de sorte que le dispositif distant (60) peut être opéré pour recevoir le signal de sortie transmis.
